# EUROPEAN PATENT SPECIFICATION

(11) **EP 2 983 069 B1**
(45) Date of publication and mention of the grant of the patent: **19.12.2018**
(21) Application number: 14776469.0
(22) Date of filing: 17.01.2014
(51) Int. Cl.: G06F 3/0354, A61B 34/37, A61B 1/00, G06F 3/0362

(54) **OPERATION INPUT DEVICE AND MASTER-SLAVE SYSTEM**
OPERATIONSEINGABEVORRICHTUNG UND MASTER-SLAVE-SYSTEM
DISPOSITIF D'ENTRÉE D'OPÉRATION ET SYSTÈME MAÎTRE-ESCLAVE

(30) Priority: 27.03.2013 US 201361805722 P
(43) Date of publication of application: 10.02.2016
(73) Proprietor: Olympus Corporation, Hachioji-shi, Tokyo 192-8507 (JP)
(72) Inventor: YAMAMURA, Nahoko, Hachioji-shi, Tokyo 192-8507 (JP)
(74) Representative: Gunzelmann, Rainer
(86) International application number: PCT/JP2014/050797
(87) International publication number: WO 2014/156229

(56) References cited:
- EP-A1- 2 060 221
- EP-A1- 2 108 327
- WO-A1-2008/093455
- JP-A- 2003 131 804
- JP-A- 2012 203 440
- JP-A- 2012 203 440
- US-A1- 2009 303 180
- US-A1- 2010 256 558

## Description

### {Technical Field}

The present invention relates to an operation input device and a master-slave system.

### {Background Art}

Conventionally, as a master input device of a master-slave system, a device which is similar in shape to the slave device is known (e.g., see PTL 1).

This master input device has a plurality of joints, and in proportion to a turning operation input for the joints, corresponding curving portions of the slave device make a curving motion.

### {Citation List}

### {Patent Literature}

{PTL 1} The Publication of Japanese Patent No. 4608601

US 2009/303180 A1 concerns a computing device for providing navigation on a display. The computing device includes a first input device that provides navigation on the display screen in two dimensions. When actuated, the first input device may cause a pointing device to move in two dimensions, such as in the X-Y directions. Also, a second input device is provided that is configured to provide navigation on the display screen in one dimension. The second input device provides movement in the vertical or horizontal direction, and is situated around the first input device and is configured to be physically turned around the first input device.

EP 2 060 221 A1 concerns a medical device control system formed of a medical device including an insertion member inserted into the subject's body, and a magnetic field response portion disposed within the insertion member for generating torque in response to the magnetic field applied from outside the subject's body, a direction detection unit that detects an insertion direction of the insertion member, a user interface through which the information with respect to the control of the insertion direction is input and output, a magnetic field generation portion that generates a magnetic field that directs the insertion member to a control direction, and a user interface control unit that controls the user interface based on a discordance between the control direction and the insertion direction.

EP 2 108 327 A1 relates to an endoscopic surgical apparatus wherein treatment is performed under an endoscopic observation using an accessory inserted into a cavity in the body through a channel of the endoscope.

US 2010/256558 A1 relates to a robotic catheter system and method for automated control of a catheter and related components.

### {Summary of Invention}

### {Technical Problem}

However, when an endoscope is moved as a slave device through operation of the master device by the master-slave system of PTL 1, since the endoscope moves precisely following the master device, even when the master device is operated erroneously in an unintended direction, the endoscope moves invariably following the master device. For example, in a case of a treatment of resecting a tumor along the inner surface of the body cavity, which requires moving the endoscope while keeping the pointed end of a treatment tool at a constant depth of the inner surface of the body cavity, an erroneous operation of the master device may cause the treatment tool to cut into an unintended depth.

The present invention has been devised in view of the above situation, and an object of the invention is to provide an operation input device and a master-slave system which can suppress unintended motion of the slave device due to an erroneous operation of the master device.

### {Solution to Problem}

In order to achieve the above object, the present invention provides the following solutions.

The invention is as defined in claim 1. Claims 2-7 disclose exemplary embodiments. Thus, according to one aspect of the present invention, there is provided an endoscope system including the features of claim 1.

According to this aspect, the one-dimensional motion command signal is generated through operation of the one-dimensional operation unit, and the two-dimensional motion command signal is generated through operation of the two-dimensional operation unit. Since these one-dimensional operation unit and two-dimensional operation unit can be operated independently from each other, it is possible to avoid generation of the one-dimensional motion command signal during generation of the two-dimensional motion command signal.

As a result, since a total three-dimensional motion command signal is output, separately as the two-dimensional motion command signal and the one-dimensional motion command signal, it is possible to move an object to be operated in two-dimensional directions and the other one-dimensional direction separately and independently. That is, it is possible to prevent the inconvenience that the object to be operated moves unintendedly in the other one-dimensional direction due to operation of the two-dimensional operation
unit which moves the object to be operated in the two-dimensional directions.

In the above-described aspect, the two-dimensional operation unit may generate a two-dimensional motion command signal composed of a two-dimensional vector quantity. In particular, the two-dimensional operation unit may be a capacitance-type two-dimensional sensor.

Thus, it is possible to input a relative value of a two-dimensional vector quantity by the two-dimensional operation unit, and to reduce the size of the device by eliminating the need for a massive device.

Not according to the invention, the two-dimensional operation unit may generate a two-dimensional motion command signal composed of swing angles of a lever around two shafts which intersect with each other and around which the lever can swing.

Thus, an absolute value can be input by the two-dimensional operation unit, which allows more intuitive operation.

In the above-described aspect, the one-dimensional operation unit may generate a one-dimensional motion command signal composed of a one-dimensional vector quantity.

In the above-described aspect, the one-dimensional operation unit may generate a one-dimensional motion command signal composed of information on a position in one direction.

According to another aspect of the present invention, there is provided a master-slave system according to claim 6.

According to this aspect, the one-dimensional motion command signal is generated through operation of the one-dimensional operation unit, and the two-dimensional motion command signal is generated through operation of the two-dimensional operation unit. Since these one-dimensional operation unit and two-dimensional operation unit can be operated independently from each other, it is possible to avoid generation of the one-dimensional motion command signal during generation of the two-dimensional motion command signal.

As a result, since a total three-dimensional motion command signal is output, separately as the two-dimensional motion command signal and the one-dimensional motion command signal, it is possible to move the slave device in two-dimensional directions and the other one-dimensional direction separately and independently. That is, it is possible to prevent the inconvenience that the slave device moves unintendedly in the other one-dimensional direction due to operation of the two-dimensional operation unit which moves the slave device in the two-dimensional directions.

According to yet another aspect of the present invention, there is provided a master-slave system according to claim 7.

According to this aspect, the slave device can be turned easily by the two-dimensional operation unit constituted by the capacitance-type two-dimensional sensor. In this regard, since the turning motion is made around the axis orthogonal to the specified plane, it is possible to prevent the inconvenience that the slave device moves unintendedly in a direction intersecting with the specified plane.

### {Advantageous Effects of Invention}

According to the present invention, an effect is achieved that unintended motion of the slave device due to an erroneous operation of the master device can be suppressed.

### {Brief Description of Drawings}

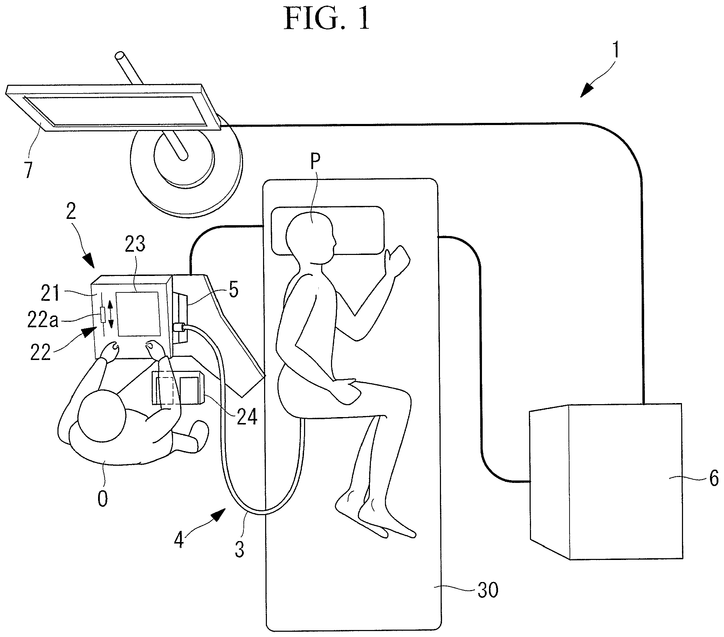

{Fig. 1}
   Fig. 1 is an overall configurational view showing a master-slave system according to one embodiment of the present invention.
{Fig. 2}
   Fig. 2 is a perspective partial view illustrating a coordinate system fixed at the leading end of an endoscope as a slave device of the master-slave system of Fig. 1.
{Fig. 3}
   Fig. 3(a) and Fig. 3(b) are views respectively showing an operation of a two-dimensional operation unit and a corresponding motion of the endoscope in an operation input device according to one embodiment of the present invention which is included in the master-slave system of Fig. 1.
{Fig. 4}
   Fig. 4(a) to Fig. 4(c) are views respectively illustrating an operation of the two-dimensional operation unit, a motion of the endoscope, and a change in a display unit, in a modified example of the operation input device of Fig. 3.
{Fig. 5}
   Fig. 5(a) to Fig. 5(c) are views respectively illustrating another operation of the two-dimensional operation unit, a motion of the endoscope, and a change in the display unit, of the operation input device of Fig. 4.
{Fig. 6}
   Fig. 6(a) to Fig. 6(c) are views respectively illustrating yet another operation of the two-dimensional operation unit, a motion of the endoscope, and a change in the display unit, of the operation input device of Fig. 4.
{Fig. 7}
   Fig. 7 is a perspective view showing another modified example of the operation input device of Fig. 3.
{Fig. 8}
   Fig. 8 is a perspective view showing a modified example of the operation input device of Fig. 3 not according to the invention.
{Fig. 9}
   Fig. 9 is a perspective view showing a one-dimensional operation unit in still another modified example of the operation input device of Fig. 3.

### {Description of Embodiments}

In the following, a master device (operation input device) 2 and a master-slave system 1 according to one embodiment of the present invention will be described with reference to the drawings.

As shown in Fig. 1, the master-slave system 1 according to this embodiment is an endoscope system, and includes the master device 2 operated by an operator O, an endoscope 4 as a slave device, a driving unit 5 which drives the endoscope 4, a control unit 6 which controls the driving unit 5, and a display unit 7 which displays images acquired by the endoscope 4.

The endoscope 4 has a flexible insert part 3 which is inserted into the body of a patient P, for example, into a soft organ such as the large intestine.

The driving unit 5 drives an insertion motion of the insert part 3 of the endoscope 4, a curving motion of the leading end of the insert part 3, a twisting motion of the insert part 3, etc., at the base end side of the insert part 3.

As shown in Fig. 1, the master device 2 according to this embodiment has an operation console (a base member) 21 fixed on a floor, a one-dimensional operation unit 22 and a two-dimensional operation unit 23 operated by the operator O, and a foot switch 24 disposed on the floor.

As shown in Fig. 1, an assistant (not shown) puts the patient P on an operating table 30 by the side of which the operation unit 2 is disposed, and performs proper treatments such as disinfection and anesthesia.

The operator O instructs the assistant to introduce the insert part 3 of the endoscope 4 from the anus into the large intestine of the patient P. The operator O appropriately curves curving portions 3a, 3b of the insert part 3, so as to move the leading end of the endoscope 4, by operating the one-dimensional operation unit 22 and the two-dimensional operation unit 23.

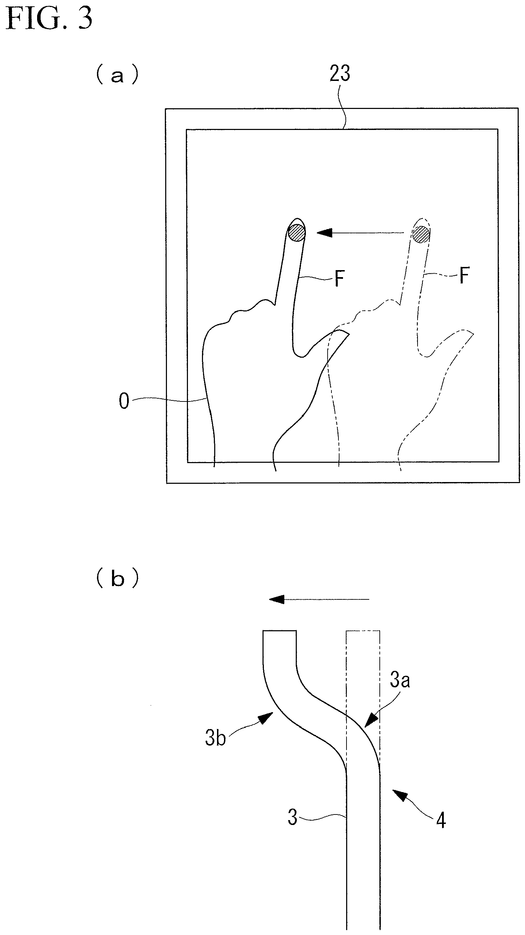

The two-dimensional operation unit 23 is a capacitance-type two-dimensional sensor provided on the operation console 21, and as shown in Fig. 3, when a finger F is slid along the flat panel surface, the two-dimensional operation unit 23 detects the shift amount and the shift direction as a two-dimensional vector quantity and outputs it as a motion command signal. That is, according to the two-dimensional operation unit 23, each time it is operated, a relative value of a two-dimensional vector quantity can be input as information on the shift amount and the shift direction relative to the current position.

The one-dimensional operation unit 22 is, for example, a slider 22a which can slide in one direction, and detects the position of the slider 22a and outputs it as a motion command signal. That is, according to the one-dimensional operation unit 21, a one-dimensional absolute value can be input by changing the output according to the position of the slider 22a.

Upon receiving the motion command signals output from the one-dimensional operation unit 22 and the two-dimensional operation unit 23, the control unit 6 converts the signals into motion command signals for the curving portions 3a, 3b of the insert part 3 of the endoscope 4 which is the slave device, and outputs the signals to the driving unit 5.

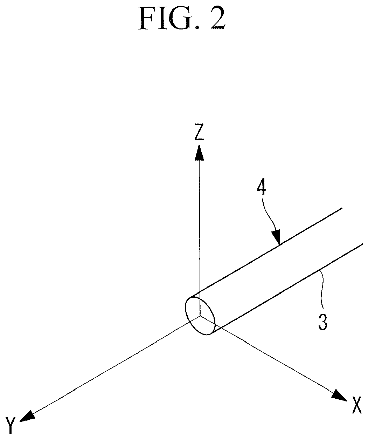

In this case, on the basis of the motion command signal output from the two-dimensional operation unit 23, the control unit 6 generates a motion command signal which activates the curving portions 3a, 3b so that the leading end of the endoscope 4 shifts within an X-Y plane (specified plane) in a coordinate system fixed at the leading end of the endoscope 4 as shown in Fig. 2.

Moreover, on the basis of the motion command signal output from the one-dimensional operation unit 22, the control unit 6 generates a motion command signal which activates the curving portions 3a, 3b so that the leading end of the endoscope 4 shifts in a Z-direction which is orthogonal to the X-Y plane as shown in Fig. 2.

The working of the master device 2 and the master-slave system 1 according to this embodiment thus configured will be described below.

To observe and treat the inside of the body cavity using the endoscope 4 with the insert part 3 of the endoscope 4 inserted inside the body cavity, the operator O operates the two-dimensional operation unit 23 and the one-dimensional operation unit 22 of the master device 2 so as to shift the leading end of the endoscope 4, while observing the state of the inside of the body cavity imaged by the endoscope 4 at the display unit 7.

Since the operation direction along the flat panel surface of the two-dimensional operation unit 23 is associated by the control unit 6 with the X-Y coordinate plane fixed at the leading end of the endoscope 4, the operator O can shift the leading end of the endoscope 4 along the X-Y coordinate plane in the same direction as the motion direction of the finger F as shown in Fig. 3(b), by performing an operation of sliding the finger F on the panel surface of the two-dimensional operation unit 23 as shown in Fig. 3(a).

Since the operation direction of the slider 22a of the one-dimensional operation unit 22 is associated by the control unit 6 with the direction orthogonal to the X-Y coordinate plane, namely, with the Z-direction, of the endoscope 4, the operator O can shift the leading end of the endoscope 4 in the Z-direction by performing an operation of sliding the slider 22a of the one-dimensional operation unit 22.

In this case, according to the master device 2 of this embodiment, it is possible to combine an operation of the one-dimensional operation unit 22 and an operation of the two-dimensional operation unit 23 so as to shift the leading end of the endoscope 4 three-dimensionally to an arbitrary position. With the master device 2 according to this embodiment, since the one-dimensional operation unit 22 and the two-dimensional operation unit 23 are provided separately and independently from each other, unless the one-dimensional operation unit 22 is operated during operation of the two-dimensional operation unit 23, the shift trajectory of the leading end of the endoscope 4 can be limited within the X-Y coordinate plane.

One advantage is that, for example, when performing resection of a tumor inside the body cavity, by presetting the X-Y plane at a position of a predetermined depth from the inner surface of the body cavity, it is possible, even in the event of an erroneous operation, to prevent a treatment tool at the leading end of the endoscope 4 from traveling in the depth direction as long as only the two-dimensional operation unit 23 is operated.

In this embodiment, it has been described that the shift direction of the finger F in the two-dimensional operation unit 23 and the shift direction of the leading end of the insert part 3 of the endoscope 4 are the same direction as shown in Fig. 3(a). Thus, the insert part 3 can be shifted intuitively through the movement of the finger F.

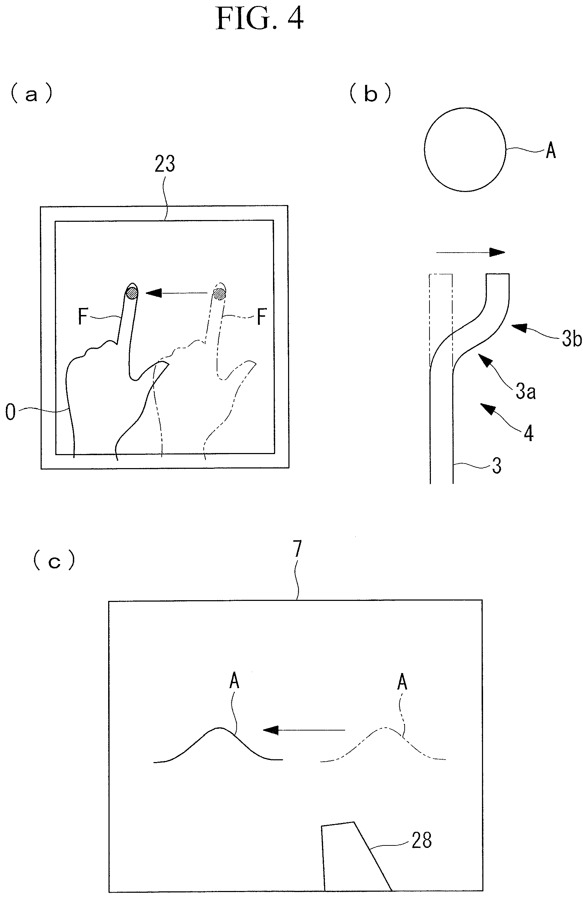

Instead of this, the insert part 3 may be shifted so that the image displayed at the display unit 7 is shifted through the movement of the finger F as shown in Fig. 4 to Fig. 6. The reference sign 28 in these drawings indicates the treatment tool which is disposed so as to protrude from the leading end of the endoscope 4 and imaged by the endoscope 4.

That is, the insert part 3 may be shifted rightward as shown in Fig. 4(b) by shifting the finger F leftward on the panel surface of the two-dimensional operation unit 23 as shown in Fig. 4(a) so that an object A displayed in the display unit 7 is shifted leftward, which is the same direction as the shift direction of the finger F, as shown in Fig. 4(c). When the finger F is shifted rightward, the insert part 3 should be shifted leftward.

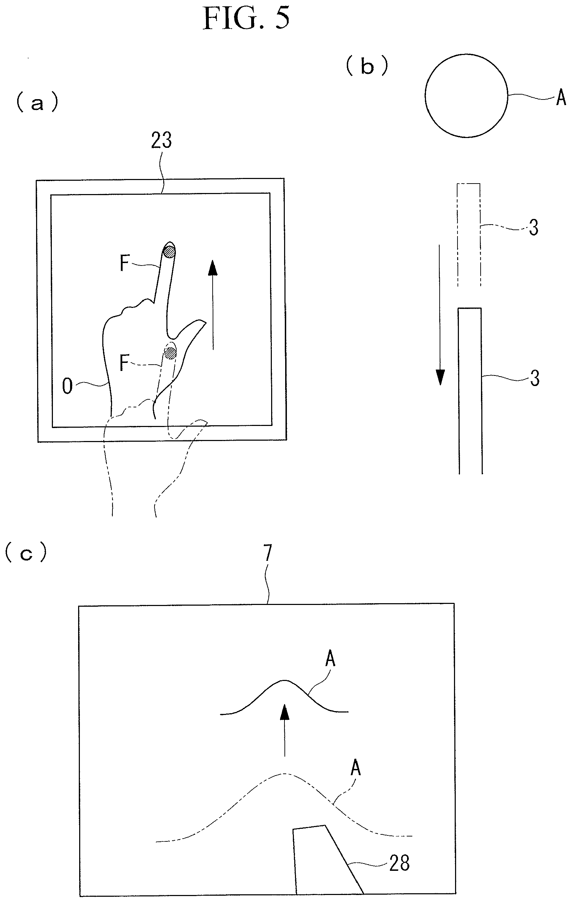

Moreover, the insert part 3 may be shifted toward the near side as shown in Fig. 5(b) by shifting the finger F forward on the panel surface of the two-dimensional operation unit 23 as shown in Fig. 5(a) so that the object A displayed in the display unit 7 is shifted toward the far side, which is the same direction as the shift direction of the finger F, as shown in Fig. 5(c). When the finger F is shifted toward the near side, the insert part 3 should be shifted toward the far side.

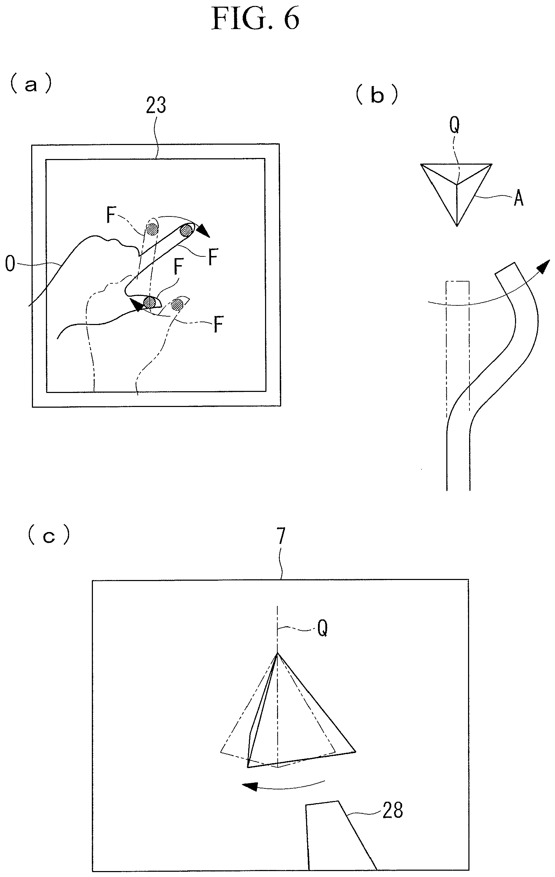

Furthermore, the insert part 3 may be turned around an axis Q, which is disposed at a predetermined distance from the leading end of the insert part 3 and extends along the Z-axis direction, in the opposite direction (counterclockwise direction) from the movement of the finger F as shown in Fig. 6(b) by performing an operation of shifting two fingers F (e.g., the forefinger and the thumb) in opposite directions (e.g., shifting the forefinger rightward and shifting the thumb leftward) and turning the fingers F in one direction (e.g., clockwise direction) on the panel surface of the two-dimensional operation unit 23 as shown in Fig. 6(a) so that the object A displayed in the display unit 7 turns around the axis Q (in the clockwise direction) as shown in Fig. 6(c). When the fingers F are turned in the counterclockwise direction, the insert part 3 should be turned in the clockwise direction.

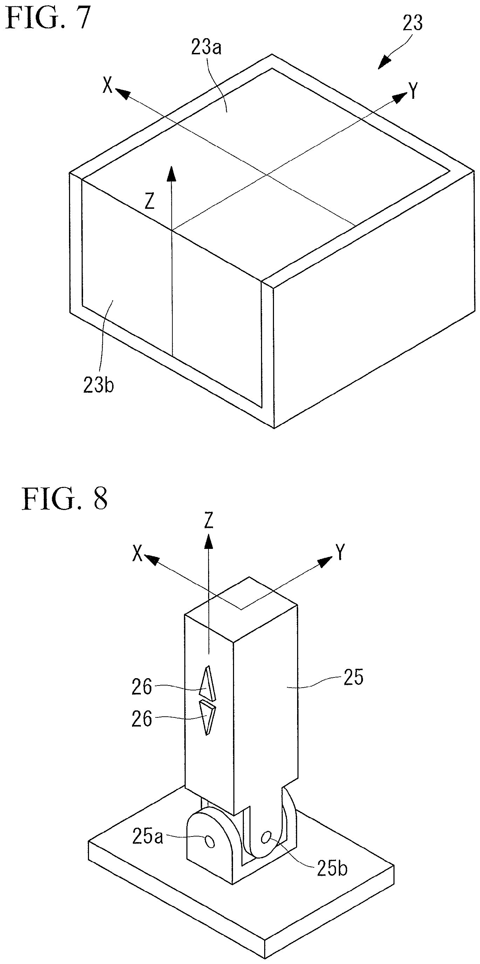

While in this embodiment the capacitance-type two-dimensional sensor as the two-dimensional operation unit 23 and the uniaxial slider 22a as the one-dimensional operation unit 22 have been illustrated, as shown in Fig. 7, another operation device may be adopted which has a capacitance-type one-dimensional sensor 23b, as the one-dimensional operation unit 22, on a surface other than the surface of a capacitance-type two-dimensional sensor 23a.

As shown in Fig. 8, not according to the invention, a lever 25 which can swing around two shafts 25a, 25b may be adopted as the two-dimensional operation unit 23, and a switch 26 provided on a side surface of the lever 25 may be adopted as the one-dimensional operation unit. The insert part 3 can be moved within the X-Y plane through operation of the lever 25, and can be moved in the Z-axis direction through operation of the switch 26.

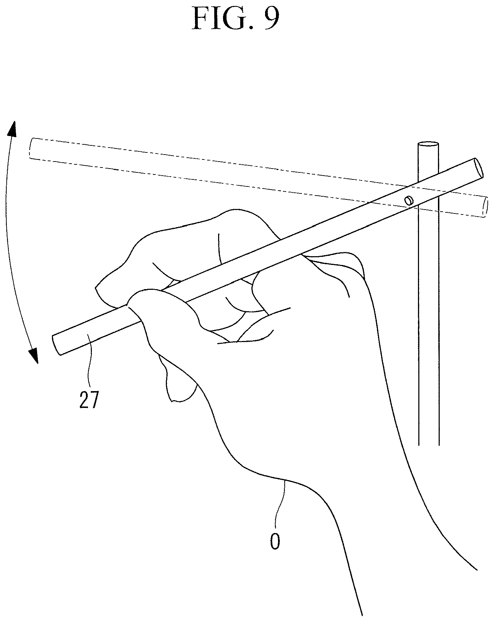

Instead of the slider 22a, as shown in Fig. 9, a lever 27 which swings in uniaxial direction may be adopted as the one-dimensional operation unit 22. This allows more intuitive operation.

While in this embodiment the two-dimensional operation by the two-dimensional operation unit 23 is associated with motions within the X-Y plane of the insert part 3 of the endoscope 4, instead of this, the two-dimensional operation by the two-dimensional operation unit 23 may be associated with motions within the X-Z plane or the Y-Z plane. Alternatively, the two-dimensional operation by the two-dimensional operation unit 23 may be associated with motions within a predefined arbitrary plane other than these planes. Furthermore, the motions are not limited to motions within a plane, and the two-dimensional operation by the two-dimensional operation unit 23 may be associated with motions within a predefined curved plane.

Not according to the invention, instead of the capacitance-type two-dimensional sensor, a two-dimensional operation unit 23 like a swinging lever may be adopted which inputs a relative value by generating a shift direction command from a swing angle and generating a shift amount from an input time.

### {Reference Signs List}

- F: Finger
- P: Operator
- 1: Master-slave system
- 2: Master device (operation input device)
- 4: Endoscope (slave device)
- 22: One-dimensional operation unit
- 23: Two-dimensional operation unit
- 25: Lever
- 25a, 25b: shaft

## Claims

1. An endoscope system comprising:
a two-dimensional operation unit (23) which can generate a two-dimensional motion command signal, wherein the two-dimensional operation unit (23) has a flat panel surface; and
a control unit (6) configured to receive the signal from the two-dimensional operation unit (23), and output a motion command signal to a driving unit (5) which is configured to curve a curving portion (3a, 3b) provided in an insert part (3) of an endoscope (4), wherein the motion command signal is based on the received signal from the two-dimensional operation unit (23), and is for curving the curving portion (3a, 3b),
**characterized by**
a one-dimensional operation unit (22) which can generate a one-dimensional motion command signal, wherein
the one-dimensional operation unit (22) and the two-dimensional operation unit (23) can be operated independently from each other, and
the control unit (6) is configured to output a motion command signal to curve the curving portion (3a, 3b) leftward when a finger (F) is moved rightward on the flat panel surface, and output a motion command signal to curve the curving portion (3a, 3b) rightward when the finger (F) is moved leftward on the flat panel surface.

2. The endoscope system according to claim 1, wherein the two-dimensional operation unit (23) is configured to generate a two-dimensional motion command signal composed of a two-dimensional vector quantity.

3. The endoscope system according to claim 2, wherein the two-dimensional operation unit (23) is a capacitance-type two-dimensional sensor.

4. The endoscope system according to any one of claims 1 to 3, wherein the one-dimensional operation unit (22) is configured to generate a one-dimensional motion command signal composed of a one-dimensional vector quantity.

5. The endoscope system according to any one of claims 1 to 3, wherein the one-dimensional operation unit (22) is configured to generate a one-dimensional motion command signal composed of information on a position in one direction.

6. A master-slave system (1) comprising:
the endoscope system according to any one of claims 1 to 5; and
an endoscope (4) which is configured to be driven on the basis of a motion command signal generated by the operation input device (22, 23), wherein
the endoscope (4) is configured to be shifted within a two-dimensional specified plane, which is previously specified, according to a two-dimensional motion command signal generated by the two-dimensional operation unit (23), and is configured to be shifted in a direction intersecting with the specified plane according to a one-dimensional motion command signal generated by the one-dimensional operation unit (22).

7. A master-slave system (1) comprising:
the endoscope system according to claim 3; and
an endoscope (4) which is configured to be driven on the basis of a motion command signal generated by the operation input device (22, 23), wherein
the endoscope (4) is configured to be shifted within a two-dimensional specified plane, which is previously specified, according to a two-dimensional motion command signal generated by the capacitance-type two-dimensional sensor, and is configured to be shifted in a direction intersecting with the specified plane according to a one-dimensional motion command signal generated by the one-dimensional operation unit (22), and additionally, the endoscope (4) is configured to be turned around a predetermined axis orthogonal to the specified plane through an operation of shifting two fingers in opposite directions while holding the fingers in contact with the capacitance-type two-dimensional sensor at a distance from each other.

## Patentansprüche

1. Endoskopsystem, umfassend:
eine zweidimensionale Betätigungseinheit (23), die ein zweidimensionales Bewegungsbefehlssignal generieren kann, wobei die zweidimensionale Betätigungseinheit (23) eine flache Plattenfläche aufweist; und
eine Steuereinheit (6), die konfiguriert ist, um das Signal von der zweidimensionalen Betätigungseinheit (23) zu empfangen und um ein Bewegungsbefehlssignal an eine Antriebseinheit (5) auszugeben, die konfiguriert ist, um einen Biegeabschnitt (3a, 3b), der in einem Einsetzteil (3) eines Endoskops (4) bereitgestellt wird, zu biegen, wobei das Bewegungsbefehlssignal auf dem empfangenen Signal von der zweidimensionalen Betriebseinheit (23) basiert und zum Biegen des Biegeabschnitts (3a, 3b) gedacht ist,
**gekennzeichnet durch**
eine eindimensionale Betätigungseinheit (22), die ein eindimensionales Bewegungsbefehlssignal generieren kann, wobei die eindimensionale Betätigungseinheit (22) und die zweidimensionale Betätigungseinheit (23) unabhängig voneinander betätigt werden können, und die Steuereinheit (6) konfiguriert ist, um ein Bewegungsbefehlssignal auszugeben, um den Biegeabschnitt (3a, 3b) nach links zu biegen, wenn ein Finger (F) auf der flachen Plattenfläche nach rechts bewegt wird, und um ein Bewegungsbefehlssignal auszugeben, um den Biegeabschnitt (3a, 3b) nach rechts zu biegen, wenn der Finger (F) auf der flachen Plattenfläche nach links bewegt wird.

2. Endoskopsystem nach Anspruch 1, wobei die zweidimensionale Betätigungseinheit (23) konfiguriert ist, um ein zweidimensionales Bewegungsbefehlssignal zu generieren, das aus einer zweidimensionalen Vektorgröße besteht.

3. Endoskopsystem nach Anspruch 2, wobei die zweidimensionale Betätigungseinheit (23) ein zweidimensionaler kapazitiver Sensor ist.

4. Endoskopsystem nach einem der Ansprüche 1 bis 3, wobei die eindimensionale Betätigungseinheit (22) konfiguriert ist, um ein eindimensionales Bewegungsbefehlssignal zu generieren, das aus einer eindimensionalen Vektorgröße besteht.

5. Endoskopsystem nach einem der Ansprüche 1 bis 3, wobei die eindimensionale Betätigungseinheit (22) konfiguriert ist, um ein eindimensionales Bewegungsbefehlssignal zu generieren, das aus Information über eine Position in einer Richtung besteht.

6. Master-Slave-System (1), umfassend:
das Endoskopsystem nach einem der Ansprüche 1 bis 5; und
ein Endoskop (4), das konfiguriert ist, um auf der Grundlage eines Bewegungsbefehlssignals angetrieben zu werden, das durch die Betätigungseingabevorrichtung (22, 23) generiert wird, wobei das Endoskop (4) konfiguriert ist, um in einer zweidimensionalen vorgeschriebenen Ebene, die zuvor vorgegeben wird, gemäß einem zweidimensionalen Bewegungsbefehlssignal, das durch die zweidimensionale Betätigungseinheit (23) generiert wird, verschoben zu werden, und konfiguriert ist, um in einer Richtung, die sich mit der vorgegeben Ebene schneidet, gemäß einem eindimensionalen Bewegungsbefehlssignal, das durch die eindimensionale Betätigungseinheit (22) generiert wird, verschoben zu werden.

7. Master-Slave-System (1), umfassend:
das Endoskopsystem nach Anspruch 3; und
ein Endoskop (4), das konfiguriert ist, um auf der Grundlage eines Bewegungsbefehlssignals, das durch die Betätigungseingabevorrichtung (22, 23) generiert wird, angetrieben zu werden, wobei das Endoskop (4) konfiguriert ist, um in einer zweidimensionalen vorgeschriebenen Ebene, die zuvor vorgegeben wird, gemäß einem zweidimensionalen Bewegungsbefehlssignal, das durch den zweidimensionalen kapazitiven Sensor generiert wird, verschoben zu werden, und konfiguriert ist, um in einer Richtung, die sich mit der vorgegebenen Ebene schneidet, gemäß einem eindimensionalen Bewegungsbefehlssignal, das durch die eindimensionale Betätigungseinheit (22) generiert wird, verschoben zu werden, und das Endoskop (4) zudem konfiguriert ist, um um eine vorbestimmte Achse herum, die zu der vorgegebenen Ebene orthogonal ist, durch eine Betätigung des Verschiebens von zwei Fingern in entgegengesetzte Richtungen, während die Finger in Kontakt mit dem zweidimensionalen kapazitiven Sensor in einem Abstand voneinander gehalten werden, gedreht zu werden.

## Revendications

1. Système d'endoscope comprenant :
une unité d'opération bidimensionnelle (23) qui peut générer un signal de commande de mouvement bidimensionnel, l'unité d'opération bidimensionnelle (23) ayant une surface de panneau plate ; et
une unité de commande (6) configurée pour recevoir le signal en provenance de l'unité d'opération bidimensionnelle (23) et délivrer en sortie un signal de commande de mouvement à une unité d'entraînement (5) qui est configurée pour courber une partie de courbure (3a, 3b) prévue dans une partie d'insert (3) d'un endoscope (4), le signal de commande de mouvement étant basé sur le signal reçu en provenance de l'unité d'opération bidimensionnelle (23), et étant prévue pour courber la partie de courbure (3a, 3b),
**caractérisé par**
une unité d'opération unidimensionnelle (22) qui peut générer un signal de commande de mouvement unidimensionnel,
l'unité d'opération unidimensionnelle (22) et l'unité d'opération bidimensionnelle (23) pouvant être actionnées indépendamment l'une de l'autre, et
l'unité de commande (6) étant configurée pour délivrer en sortie un signal de commande de mouvement pour courber la partie de courbure (3a, 3b) vers la gauche lorsqu'un doigt (F) est déplacé vers la droite sur la surface de panneau plate, et délivrer en sortie un signal de commande de mouvement pour courber la partie de courbure (3a, 3b) vers la droite lorsque le doigt (F) est déplacé vers la gauche sur la surface de panneau plate.

2. Système d'endoscope selon la revendication 1, dans lequel l'unité d'opération bidimensionnelle (23) est configurée pour générer un signal de commande de mouvement bidimensionnel composé d'une quantité vectorielle bidimensionnelle.

3. Système d'endoscope selon la revendication 2, dans lequel l'unité d'opération bidimensionnelle (23) est un capteur bidimensionnel de type capacitif.

4. Système d'endoscope selon l'une quelconque des revendications 1 à 3, dans lequel l'unité d'opération unidimensionnelle (22) est configurée pour générer un signal de commande de mouvement unidimensionnel composé d'une quantité vectorielle bidimensionnelle.

5. Système d'endoscope selon l'une quelconque des revendications 1 à 3, dans lequel l'unité d'opération unidimensionnelle (22) est configurée pour générer un signal de commande de mouvement unidimensionnel composé d'informations relatives à une position dans une direction.

6. Système maître-esclave (1) comprenant :
le système d'endoscope selon l'une quelconque des revendications 1 à 5 ; et
un endoscope (4) qui est configuré pour être entraîné sur la base d'un signal de commande de mouvement généré par le dispositif d'entrée d'opération (22, 23),
l'endoscope (4) étant configuré pour être déplacé dans un plan spécifié bidimensionnel, qui est spécifié au préalable, en fonction d'un signal de commande de mouvement bidimensionnel généré par l'unité d'opération bidimensionnelle (23), et étant configuré pour être déplacé dans une direction coupant le plan spécifié en fonction d'un signal de commande de mouvement unidimensionnel généré par l'unité d'opération unidimensionnelle (22).

7. Système maître-esclave (1) comprenant :
le système d'endoscope selon la revendication 3 ; et
un endoscope (4) qui est configuré pour être entraîné sur la base d'un signal de commande de mouvement généré par le dispositif d'entrée d'opération (22, 23),
l'endoscope (4) étant configuré pour être déplacé dans un plan spécifié bidimensionnel, qui est spécifié au préalable, en fonction d'un signal de commande de mouvement bidimensionnel généré par le capteur bidimensionnel capacitif, et étant configuré pour être déplacé dans une direction coupant le plan spécifié en fonction d'un signal de commande de mouvement unidimensionnel généré par l'unité d'opération unidimensionnelle (22) et, de plus, l'endoscope (4) étant configuré pour être tourné autour d'un axe prédéterminé orthogonal au plan spécifié par l'intermédiaire d'une opération de déplacement de deux doigts dans des directions opposées tout en maintenant les doigts en contact avec le capteur bidimensionnel de type capacitif à une certaine distance l'un de l'autre.
